# EUROPÄISCHE PATENTSCHRIFT

(11) **EP 2 779 895 B1**
(45) Veröffentlichungstag und Bekanntmachung des Hinweises auf die Patenterteilung: **11.12.2019**
(21) Anmeldenummer: 12798139.7
(22) Anmeldetag: 27.10.2012
(51) Int. Cl.: A61B 5/053

(54) **VERFAHREN UND VORRICHTUNG ZUR ERMITTLUNG BIO-IMPEDANZDATEN EINER PERSON**
METHOD AND DEVICE FOR MEASURING BIO-IMPEDANCE DATA OF A PERSON
PROCÉDÉ ET DISPOSITIF DE DÉTERMINATION DE DONNÉES DE BIO-IMPÉDANCE D'UNE PERSONNE

(30) Priorität: 15.11.2011 DE 102011118811
(43) Veröffentlichungstag der Anmeldung: 24.09.2014
(73) Patentinhaber: seca ag, 4153 Reinach BL 1 (CH)
(72) Erfinder: VON MAYDELL, Marc-Oliver, 22337 Hamburg (DE); VOGEL, Frederik, 20149 Hamburg (DE)
(74) Vertreter: Klickow & Wetzel PartGmbB
(86) Internationale Anmeldenummer: PCT/DE2012/001061
(87) Internationale Veröffentlichungsnummer: WO 2013/071908

(56) Entgegenhaltungen:
- CA-A1- 2 269 173
- US-A1- 2004 059 242
- US-A1- 2005 080 353
- US-A1- 2005 182 341
- US-A1- 2010 312 148
- US-B1- 6 369 337

## Beschreibung

Die Erfindung betrifft ein Verfahren zur Ermittlung von Bio-Impedanzdaten einer stehenden Person , bei dem mindestens ein Impedanzwert unter Verwendung von mindestens zwei Paaren von Elektroden für jede Hand der stehenden Person gemessen und zu einer Auswertungseinrichtung übermittelt wird und gemäß dem im Bereich mindestens einer Ausgabeeinrichtung Informationen für die Person zur Einnahme einer für die Messung der Bio-Impedanz optimalen Körperhaltung bereitgestellt werden sowie bei dem die Informationen visuell derart dargestellt werden, dass eine mindestens teilweise Erfassung einer tatsächlichen Körperhaltung der Person durchgeführt wird und bei dem die Informationen auf Basis eines Vergleiches einer tatsächlichen Körperhaltung mit einer Sollkörperhaltung ausgegeben werden.

Die Erfindung betrifft darüber hinaus eine Vorrichtung zur Ermittlung von Bio-Impedanzdaten einer stehenden Person , die zum Messen mindestens eines Bio-Impedanzwertes mindestens zwei Paare von Elektroden für jede Hand der stehenden Person aufweist, die zur Übertragung von Messwerten mit einer Auswertungseinrichtung gekoppelt sind, wobei die Auswertungseinrichtung mit einer Ausgabeeinrichtung gekoppelt ist und bei der die Ausgabeeinrichtung zur Bereitstellung von Informationen über eine optimale Körperhaltung der Person ausgebildet ist sowie bei der die Ausgabeeinrichtung zur Darstellung von visuellen Informationen derart ausgebildet ist, dass die Vorrichtung mindestens eine Erfassungseinrichtung zur Ermittlung einer tatsächlichen Körperhaltung einer Person aufweist, und bei der die Auswertungseinrichtung zur Durchführung eines Soll-Ist-Vergleiches hinsichtlich der Körperhaltung der Person ausgebildet ist.

Derartige Verfahren und Vorrichtungen werden typischerweise unter Verwendung sogenannter Body-Composition-Analyser (BCA) durchgeführt. Häufig sind derartige Vorrichtungen mit zwei Paaren Elektroden ausgestattet auf die sich die zu vermessende Person stellt. Darüber hinaus sind häufig für jede Hand der Person weitere Elektroden vorgesehen.

Bei einer Verwendung derartiger Vorrichtungen und einer Anwendung der Verfahren zeigt es sich, dass die Qualität der Messwerte wesentlich durch die konkrete Körperhaltung der Person beeinflusst wird. Bei einer ungünstigen Körperhaltung können Messwerte auftreten, die zu falschen oder ungenauen Messergebnissen führen.

Aus der US 2010/312148 A1 ist bereits ein Verfahren und eine Vorrichtung zur Ermittlung von Bio-Impedanzdaten einer Person mit einer Haltungsbewertungseinrichtung bekannt. Der Impedanzwert wird unter Verwendung von mindestens zwei Elektroden gemessen und zu einer Auswertungseinrichtung übermittelt. Es wird eine Ausgabeeinrichtung zur Bereitstellung des Bewertungsergebnisses der Haltungsbewertungseinrichtung verwendet.

Aus der US 2004/059242 A1 ist ebenfalls ein Verfahren zur Ermittlung von Bio-Impedanzdaten einer Person bekannt. Auch hier werden mindestens zwei Elektroden zur Erfassung von Impedanzwerten verwendet.

Weitere Verfahren zur Ermittlung von Bio-Impedanzdaten unter Verwendung von Elektroden werden in der US 2005/080353 A1 sowie der US 6369337 B1 beschrieben.

Aus der US 2005/182341 A1 ist die Erfassung der Körperhaltung einer Person unter Verwendung einer Kamera bekannt.

In der CA 2 269 173 A1 wird ebenfalls die Erfassung der Körperhaltung einer Person unter Verwendung einer Kamera bzw. einer anderen Messeinrichtung beschrieben.

Aufgabe der vorliegenden Erfindung ist es, ein Verfahren der einleitend genannten Art derart zu verbessern, dass die Messgenauigkeit erhöht wird.

Diese Aufgabe wird erfindungsgemäß dadurch gelöst, dass die Ermittlung der Bioimpedanzdaten unter Verwendung eines Body-Composition-Analyzers (BCA) erfolgt, dass zwei Paare von Elektroden für die Füße des Benutzers verwendet sind, dass bei einer ersten Benutzung eine korrekte Positionierung für die Hand der zu vermessenden Person festgelegt und anhand dieser Daten bei einer nächsten Benutzung verglichen wird, ob der betreffende Benutzer wieder diese als bereits korrekt bekannten Elektroden kontaktiert und dass jeweils für die linke und rechte Hand der Person im Bereich der Anzeige Korrekturdarstellungen vorgesehen sind, die signalisieren, ob die jeweilige Hand stärker angehoben oder abgesenkt werden soll bzw. ob die betreffende Hand weiter links oder rechts zu positionieren ist.

Weitere Aufgabe der vorliegenden Erfindung ist es, eine Vorrichtung der einleitend genannten Art zu konstruieren, dass die Durchführung von genauen Messungen unterstützt wird.

Diese Aufgabe wird erfindungsgemäß dadurch gelöst, dass die Ermittlung der Bioimpedanzdaten unter Verwendung eines Body-Composition-Analyzers (BCA) erfolgt, dass zwei Paare von Elektroden für die Füße des Benutzers verwendet sind, dass bei einer ersten Benutzung eine korrekte Positionierung für die Hand der zu vermessenden Person festgelegt und anhand dieser Daten bei einer nächsten Benutzung verglichen wird, ob der betreffende Benutzer wieder diese als bereits korrekt bekannten Elektroden kontaktiert und dass jeweils für die linke und rechte Hand der Person im Bereich der Anzeige Korrekturdarstellungen vorgesehen sind, die signalisieren, ob die jeweilige Hand stärker angehoben oder abgesenkt werden soll bzw. ob die betreffende Hand weiter links oder rechts zu positionieren sind.

Die Verwendung einer Ausgabeeinrichtung für die Bereitstellung von Informationen über eine Körperhaltung der zu vermessenden Person ermöglicht es, der Person Hinweise zur Einnahme einer entsprechenden Körperhaltung oder zur Korrektur einer bereits eingenommenen Körperhaltung zu geben.

Dies kann beispielsweise visuell im Bereich einer Anzeige, akustisch oder durch ein optisches Markieren der zu positionierenden Körperteile erfolgen.

Eine gute Wahrnehmbarkeit der Informationen wird dadurch erleichtert, dass die Informationen visuell dargestellt werden.

Eine Optimierung der Körperhaltung wird insbesondere dadurch unterstützt, dass eine mindestens teilweise Erfassung einer tatsächlichen Körperhaltung der Person durchgeführt wird.

Eine einfache gerätetechnische Realisierung wird dadurch unterstützt, dass die Körperhaltung der Person unter Verwendung einer Kamera erfasst wird.

Gemäß einer weiteren Ausführungsform ist auch daran gedacht, dass die Körperhaltung über eine mechanische Messeinrichtung erfasst wird, insbesondere taktil. Auch kann über den Einbau von Bewegungssensoren und/oder Triangulation die Position der Handelektroden im Raum ermittelt werden (ähnlich de.wikipedia.org/wiki/Wii-Fernbedienung).

Eine nochmalige Verbesserung der Messqualität kann dadurch erreicht werden, dass die Informationen auf Basis eines Vergleiches einer tatsächlichen Körperhaltung mit einer Sollkörperhaltung ausgegeben werden.

In den Zeichnungen sind Ausführungsbeispiele der Erfindung schematisch dargestellt. Es zeigen:
- Fig. 1: Eine perspektivische teilweise Darstellung eines Handgriffes eines Body-Composition-Analysers,
- Fig. 2: eine perspektivische Darstellung einer Person, die auf einem Body-Composition-Analyser steht,
- Fig. 3A: eine schematische Darstellung einer visuellen Anzeige mit Hinweisen zur einer Korrektur der Körperhaltung,
- Fig. 3B: eine Anzeige entsprechend Fig. 3A mit einer Kennzeichnung hinsichtlich einer bereits korrekten Körperhaltung,
- Fig. 4: eine perspektivische Darstellung einer weiteren Vorrichtung, bei der eine zu vermessende Person zwei Handelektroden ergriffen hat.

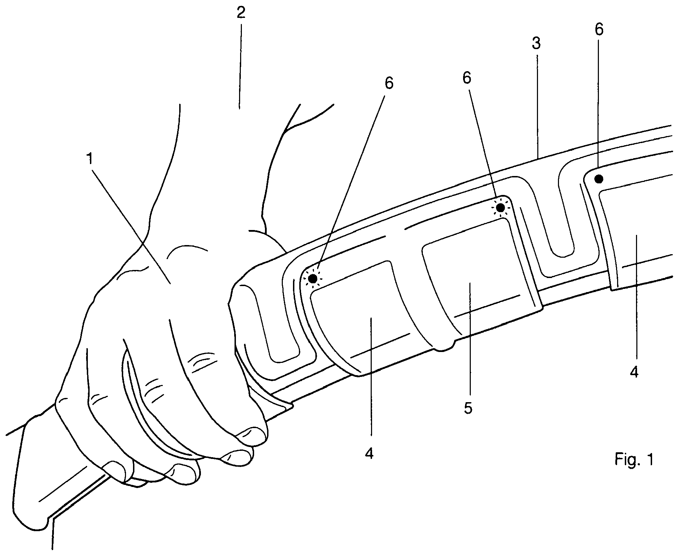

Gemäß der Darstellung in Fig. 1 ist eine Hand (1) einer zu vermessenden Person (2) im Bereich eines Handgriffes (3) einer Vorrichtung zur Ermittlung von Bio-Impedanzdaten positioniert. Im Bereich des Handgriffes (3) sind eine Mehrzahl von Elektroden (4, 5) angeordnet. Zumindest im Bereich einiger der Elektroden (4, 5) sind Leuchtanzeigen (6) angeordnet, beispielsweise lichtemittierende Dioden.

Die Leuchtanzeigen (6) können beispielsweise durch eine grüne oder rote Farbe eine korrekte oder eine fehlerhafte Positionierung der Hand (1) signalisieren.

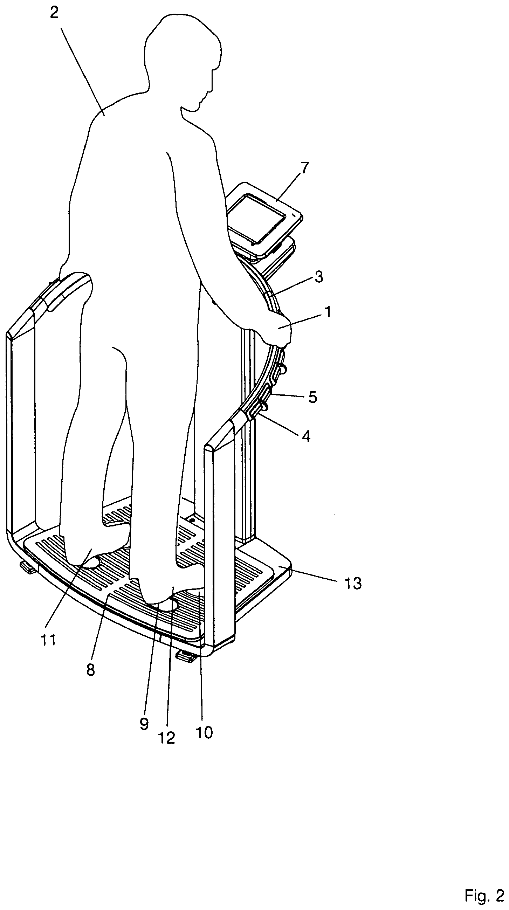

Fig. 2 zeigt eine schematische Darstellung der Person stehend auf der Vorrichtung zur Messung von Bio-Impedanzdaten. Die Person (2) hat den Handgriff (3) im Bereich einiger Elektroden (4, 5) ergriffen. In Blickrichtung vor der Person (2) ist eine Anzeige (7) zu erkennen. Die Anzeige (7) kann beispielsweise als ein Bildschirm oder LED ausgebildet sein.

Im Bereich einer Standfläche (8) sind Elektroden (9, 10) für Füße (11, 12) der Person (2) angeordnet. Im Bereich der Standfläche (8) kann eine Waage (13) zur Erfassung des Gewichtes der Person (2) positioniert sein.

Bei dem dargestellten Ausführungsbeispiel verläuft der Handgriff (3) halbkreisähnlich vergleichbar zu einer Reling. Der Handgriff (3) ist darüber hinaus relativ zur horizontalen Richtung geneigt angeordnet, um eine griffgünstige Positionierung der Elektroden (4, 5) zu unterstützen.

Entlang des Handgriffes (3) können mehrere Paare von Elektroden (4, 5) angeordnet sein. Jedes Paar gibt hierbei eine Position für die Hände des Benutzers vor.

Die richtige Körperposition des Benutzers kann beispielsweise unter Verwendung einer elektrischen Erkennung der jeweils von der Person kontaktierten Elektrodenpaare erfolgen.

Beispielsweise ist es möglich, bei einer ersten Benutzung von einer Assistenzperson eine korrekte Positionierung für die Hand der zu vermessenden Person festzulegen. Diese Position kann beispielsweise in einer elektronischen Patientenakte gespeichert werden. Bei der nächsten Benutzung wird verglichen, ob der betreffende Benutzer wieder diese als bereits korrekt bekannten Elektroden kontaktiert hat. Eine Rückmeldung an die zu vermessende Person oder die Assistenzperson kann beispielsweise dadurch erfolgen, dass im Bereich des Handgriffes (3) grüne oder rote LED (6) aufleuchten. Bei einer richtigen Positionierung würden die grünen LED und bei einer falschen Positionierung die roten LED angesteuert werden.

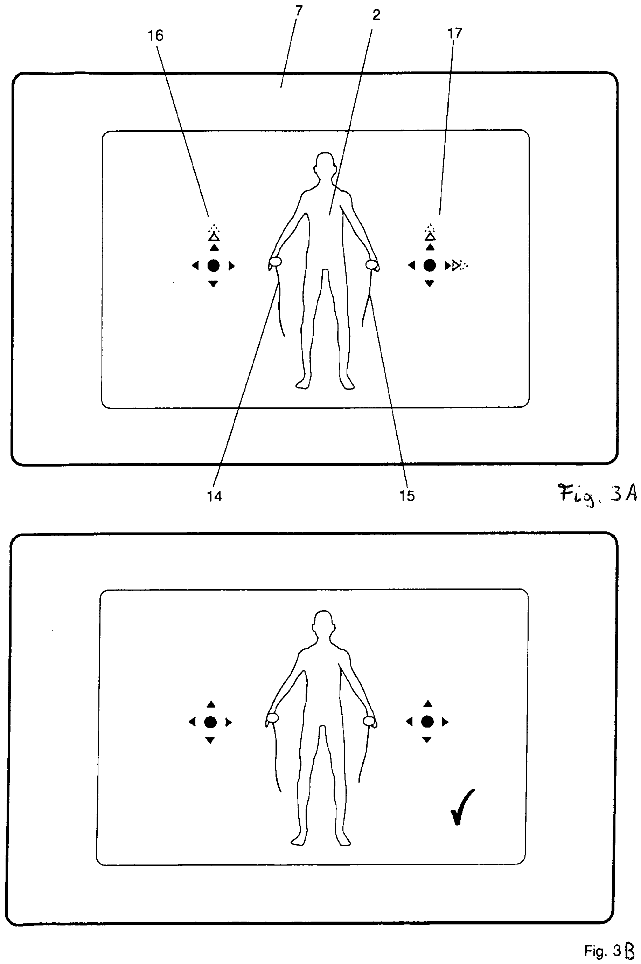

Fig. 3A zeigt eine als Display ausgebildete Anzeige (7) mit der Darstellung einer Person (2), die Handelektroden (14, 15) umgriffen hat. Jeweils für die linke und die rechte Hand der Person (2) sind im Bereich der Anzeige (7) Korrekturdarstellungen (16, 17) vorgesehen. Die Korrekturdarstellungen (16, 17) signalisieren, ob die jeweilige Hand stärker angehoben oder abgesenkt werden soll beziehungsweise ob die betreffende Hand weiter links oder rechts zu positionieren ist.

Gemäß der Darstellung in Fig. 3B wird signalisiert, dass keine Korrektur der Handposition erforderlich ist.

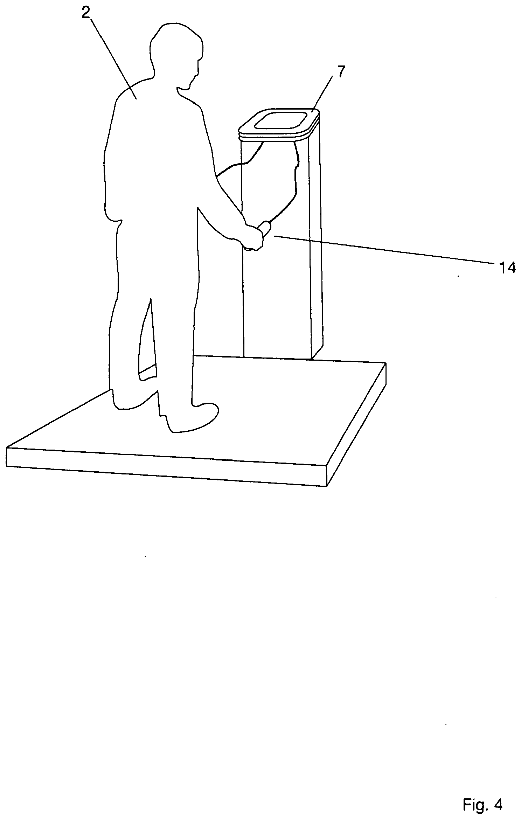

Fig. 4 veranschaulicht eine weitere Person, die im Bereich der rechten Hand eine Handelektrode (14) hält.

Gedacht ist insbesondere an die Verwendung von berührungslosen Messeinrichtungen. Eine optische Messung kann unter Verwendung einer Kamera oder ohne Verwendung einer Kamera durchgeführt werden. Einsetzbar sind beispielsweise Lichtschranken, Laserscanner oder die Durchführung einer Triangulation. Anwendbar sind auch generell elektrische Messverfahren, sowie kapazitive Messverfahren. Eine weitere Messung kann unter Verwendung von Ultraschall durchgeführt werden.

## Patentansprüche

1. Verfahren zur Ermittlung von Bio-Impedanzdaten einer stehenden Person (2), bei dem mindestens ein Bio-Impedanzwert unter Verwendung von mindestens vier Paaren von Elektroden (4, 5, 9, 10, 14, 15) für jede Hand und jeden Fuß der stehenden Person gemessen und zu einer Auswertungseinrichtung übermittelt wird und gemäß dem im Bereich mindestens einer Ausgabeeinrichtung Informationen für die Person zur Einnahme einer für die Messung der Bio-Impedanz optimalen Körperhaltung bereitgestellt werden sowie bei dem die Informationen visuell unter Verwendung einer als Display ausgebildeten Anzeige derart dargestellt werden, dass eine mindestens teilweise Erfassung einer tatsächlichen Körperhaltung der Person durchgeführt wird und bei dem die Informationen auf Basis eines Vergleiches einer tatsächlichen Körperhaltung mit einer Sollkörperhaltung ausgegeben werden, wobei die Ermittlung der Bioimpedanzdaten unter Verwendung eines Body-Composition-Analyzers (BCA) erfolgt **dadurch gekennzeichnet, dass** bei einer ersten Benutzung eine korrekte Soll-Positionierung für die Hand der zu vermessenden Person festgelegt und anhand dieser Daten bei einer nächsten Benutzung verglichen wird, ob der betreffende Benutzer wieder diese als bereits korrekt bekannten Elektroden kontaktiert und dass jeweils für die linke und rechte Hand der Person im Bereich der Anzeige Korrekturdarstellungen vorgesehen sind, die signalisieren, ob die jeweilige Hand stärker angehoben oder abgesenkt werden soll bzw. ob die betreffende Hand weiter links oder rechts zu positionieren ist.

2. Verfahren nach Anspruch 1, **dadurch gekennzeichnet, dass** die Körperhaltung der Person unter Verwendung einer Kamera erfasst wird.

3. Verfahren nach Anspruch 1, **dadurch gekennzeichnet, dass** die Körperhaltung über eine mechanische Messeinrichtung erfasst wird.

4. Verfahren nach Anspruch 1, **dadurch gekennzeichnet, dass** die Körperhaltung über eine elektrische und/oder kapazitive Messeinrichtung erfasst wird.

5. Verfahren nach Anspruch 1, **dadurch gekennzeichnet, dass** die Körperhaltung über eine Ultraschall-Messeinrichtung erfasst wird.

6. Verfahren nach Anspruch 1, **dadurch gekennzeichnet, dass** die Körperhaltung über eine optische Messeinrichtung erfasst wird.

7. Verfahren nach Anspruch 1, **dadurch gekennzeichnet, dass** die Körperhaltung über eine berührungslose Messeinrichtung erfasst wird.

8. Verfahren nach einem der Ansprüche 1 bis 7, **dadurch gekennzeichnet, dass** im Bereich mindestens einer der Elektroden eine Information zu einer Körperhaltung von mindestens einer Leuchtanzeige signalisiert wird.

9. Vorrichtung zur Ermittlung von Bio-Impedanzdaten einer stehenden Person (2), die zum Messen mindestens eines Bio-Impedanzwertes mindestens vier Paare von Elektroden (4, 5, 9, 10, 14, 15) für jede Hand und jeden Fuß der stehenden Person aufweist, die zur Übertragung von Messwerten mit einer Auswertungseinrichtung gekoppelt sind, wobei die Auswertungseinrichtung mit einer Ausgabeeinrichtung gekoppelt ist und bei der die Ausgabeeinrichtung zur Bereitstellung von Informationen über eine optimale Körperhaltung der Person (2) ausgebildet ist sowie bei der die Ausgabeeinrichtung zur Darstellung von visuellen Informationen als Anzeige ein Display aufweist, dass die Vorrichtung mindestens eine Erfassungseinrichtung zur Ermittlung einer tatsächlichen Körperhaltung einer Person aufweist, und bei der die Auswertungseinrichtung zur Durchführung eines Soll-Ist-Vergleiches hinsichtlich der Körperhaltung der Person (2) ausgebildet ist, wobei die Ermittlung der Bioimpedanzdaten unter Verwendung eines Body-Composition-Analyzers (BCA) erfolgt, **dadurch gekennzeichnet, dass** bei einer ersten Benutzung eine korrekte Soll-Positionierung für die Hand der zu vermessenden Person gespeichert und anhand dieser Daten bei einer nächsten Benutzung verglichen wird, ob der betreffende Benutzer wieder diese als bereits korrekt bekannten Elektroden kontaktiert und dass jeweils für die linke und rechte Hand der Person im Bereich der Anzeige Korrekturdarstellungen vorgesehen sind, die signalisieren, ob die jeweilige Hand stärker angehoben oder abgesenkt werden soll bzw. ob die betreffende Hand weiter links oder rechts zu positionieren ist.

10. Vorrichtung nach Anspruch 9, **dadurch gekennzeichnet, dass** die Erfassungseinrichtung als eine Kamera ausgebildet ist.

11. Vorrichtung nach Anspruch 9, **dadurch gekennzeichnet, dass** die Erfassungseinrichtung als eine mechanische Messeinrichtung ausgebildet ist.

## Claims

1. Method for ascertaining bio-impedance data of a standing person (2), in which at least one bio-impedance value is measured using at least four pairs of electrodes (4, 5, 9, 10, 14, 15) for each hand and each foot of the standing person and is transmitted to an evaluation device and according to which information for the person in respect of adopting optimal body posture for measuring the bio-impedance is provided in the region of at least one output device and in which the information is represented visually using an indication apparatus embodied as a display so that an at least partial detection of the actual body posture of the person is performed and in which the information is output on the basis of a comparison between the actual body posture and the intended body posture, wherein the bio-impedance data are ascertained using a body composition analyser (BCA), **characterized in that** correct intended positioning for the hand of the person to be measured is set during a first use and, using this data, a comparison is made during the next use as to whether the relevant user contacts these electrodes, already known as correct, again, and **in that** correction representations are provided in the region of the indication apparatus for the left and right hand, respectively, said correction representations signalling whether the respective hand should be lifted or lowered more strongly or whether the relevant hand should be positioned further to the left or to the right.

2. Method according to Claim 1, **characterized in that** the body posture of the person is captured using a camera.

3. Method according to Claim 1, **characterized in that** the body posture is captured by way of a mechanical measuring device.

4. Method according to Claim 1, **characterized in that** the body posture is captured by way of an electrical and/or capacitive measuring device.

5. Method according to Claim 1, **characterized in that** the body posture is captured by way of an ultrasonic measuring device.

6. Method according to Claim 1, **characterized in that** the body posture is captured by way of an optical measuring device.

7. Method according to Claim 1, **characterized in that** the body posture is captured by way of a contactless measuring device.

8. Method according to any one of Claims 1 to 7, **characterized in that** information relating to body posture is signalled by at least one luminous indication device in the region of at least one of the electrodes.

9. Apparatus for ascertaining bio-impedance data of a standing person (2), comprising at least four pairs of electrodes (4, 5, 9, 10, 14, 15) for each hand and each foot of the standing person for measuring at least one bio-impedance value, said electrodes being coupled to an evaluation device for the purposes of transferring measurement values, wherein the evaluation device is coupled to an output device and in which the output device is embodied so as to provide information about an optimal body posture of the person (2) and in which the output device has a display as an indication apparatus for representing visual information, in that the apparatus has at least one capturing device for ascertaining an actual body posture of a person, and in which the evaluation device is embodied to carry out actual-intended comparison in respect of the body posture of the person (2), wherein the bio-impedance data are ascertained using a body composition analyser (BCA), **characterized in that** the correct intended positioning for the hand of the person to be measured is stored during a first use and the comparison is carried out on the basis of these data during the next use as to whether the relevant person contacts these electrodes, already known as correct, again, and **in that** correction representations are provided in the region of the indication apparatus for the left and right hand, respectively, said correction representations signalling whether the respective hand should be lifted or lowered more strongly or whether the relevant hand should be positioned further to the left or right.

10. Apparatus according to Claim 9, **characterized in that** the capturing device is embodied as a camera.

11. Apparatus according to Claim 9, **characterized in that** the capturing device is embodied as a mechanical measuring device.

## Revendications

1. Procédé de détermination de données de bio-impédance d'une personne debout (2), dans lequel on mesure au moins une valeur de bio-impédance en utilisant au moins quatre paires d'électrodes (4, 5, 9, 10, 14, 15) pour chaque main et chaque pied de la personne debout et on la transmet à un dispositif d'évaluation et selon lequel on fournit dans la région d'au moins un dispositif de sortie des informations destinées à la personne afin qu'elle prenne une posture optimale pour la mesure de la bio-impédance et dans lequel on représente les informations visuellement en utilisant un affichage formé par un écran d'affichage, de telle manière que l'on effectue une détection au moins partielle d'une posture effective de la personne et dans lequel on émet les informations sur la base d'une comparaison d'une posture effective avec une posture de consigne, dans lequel on effectue la détermination de données de bio-impédance en utilisant un analyseur de la composition du corps (BCA), **caractérisé en ce que** lors d'une première utilisation on fixe un positionnement de consigne correct pour la main de la personne à mesurer et on compare à l'aide de ces données, lors d'une utilisation ultérieure, si l'utilisateur concerné contacte de nouveau ces électrodes déjà connues comme correctes et **en ce qu'**il est prévu respectivement pour la main gauche et la main droite de la personne dans la région de l'affichage des représentations de correction, qui signalent si la main respective doit être plus fortement relevée ou abaissée ou si la main respective doit être positionnée plus à gauche ou plus à droite.

2. Procédé selon la revendication 1, **caractérisé en ce que** l'on détecte la posture de la personne en utilisant une caméra.

3. Procédé selon la revendication 1, **caractérisé en ce que** l'on détecte la posture au moyen d'un dispositif de mesure mécanique.

4. Procédé selon la revendication 1, **caractérisé en ce que** l'on détecte la posture au moyen d'un dispositif de mesure électrique et/ou capacitif.

5. Procédé selon la revendication 1, **caractérisé en ce que** l'on détecte la posture au moyen d'un dispositif de mesure à ultrasons.

6. Procédé selon la revendication 1, **caractérisé en ce que** l'on détecte la posture au moyen d'un dispositif de mesure optique.

7. Procédé selon la revendication 1, **caractérisé en ce que** l'on détecte la posture au moyen d'un dispositif de mesure sans contact.

8. Procédé selon l'une quelconque des revendications 1 à 7, **caractérisé en ce que** l'on signale une information relative à une posture au moyen d'au moins un affichage lumineux dans la région d'au moins une des électrodes.

9. Dispositif de détermination de données de bio-impédance d'une personne debout (2), qui présente pour la mesure d'au moins une valeur de bio-impédance au moins quatre paires d'électrodes (4, 5, 9, 10, 14, 15) pour chaque main et chaque pied de la personne debout, qui sont couplées à un dispositif d'évaluation pour la transmission de valeurs de mesure, dans lequel le dispositif d'évaluation est couplé à un dispositif de sortie et dans lequel le dispositif de sortie est configuré pour fournir des informations relatives à une posture optimale de la personne (2) et dans lequel le dispositif de sortie présente comme affichage un écran d'affichage pour la représentation d'informations visuelles, dans lequel le dispositif présente au moins un dispositif de détection pour déterminer une posture effective d'une personne, et dans lequel le dispositif d'évaluation est configuré pour effectuer une comparaison consigne-effective en ce qui concerne la posture de la personne (2), dans lequel la détermination des données de bio-impédance est effectuée en utilisant un analyseur de la composition du corps (BCA), **caractérisé en ce que** lors d'une première utilisation on mémorise un positionnement de consigne correct pour la main de la personne à mesurer et on compare à l'aide de ces données, lors d'une utilisation ultérieure, si l'utilisateur concerné contacte de nouveau ces électrodes déjà connues comme correctes et **en ce qu'**il est prévu respectivement pour la main gauche et la main droite de la personne dans la région de l'affichage des représentations de correction, qui signalent si la main respective doit être plus fortement relevée ou abaissée ou si la main respective doit être positionnée plus à gauche ou plus à droite.

10. Dispositif selon la revendication 9, **caractérisé en ce que** le dispositif de détection est formé par une caméra.

11. Dispositif selon la revendication 9, **caractérisé en ce que** le dispositif de détection est formé par un dispositif de mesure mécanique.
